Europäisches Patentamt

European Patent Office

Office européen des brevets

(11) Publication number: **0 421 007 A1**

# EUROPEAN PATENT APPLICATION

(21) Application number: 89118366.7

(22) Date of filing: 04.10.89

(51) Int. Cl.⁵: **A61M 16/06**

(43) Date of publication of application:
**10.04.91 Bulletin 91/15**

(84) Designated Contracting States:
**AT BE CH DE ES FR GB GR IT LI LU NL SE**

(71) Applicant: **CALIFORNIA MEDICAL PRODUCTS, INC.**
**1901 Obispo Avenue**
**Long Beach California 90804(US)**

(72) Inventor: **Garth, Geoffrey C.**

334 Colorado Place
Long Beach, CA 90814(US)
Inventor: **Patterson, Charlie A.**
5322 Charing Cross
Westminister, CA 92683(US)

(74) Representative: **Goetz, Rupert, Dipl.-Ing. et al**
**Wuesthoff & Wuesthoff Patent- und**
**Rechtsanwälte Schweigerstrasse 2**
**W-8000 München 90(DE)**

(54) Resuscitation device.

(57) A resuscitation device is disclosed for delivering air or other appropriate gas or mixture of gases to the respiratory tract of a patient which can be operated using only one hand, thus leaving the other hand completely free to manipulate the patient's head and neck. The device comprises a housing (21) defining a chamber (26), a conduit (33) and mask (34) for delivering gas from the chamber (26) to the patient and manifolds (35) for filling the chamber (26) with gas. The housing (21), conduit (33) and mask (34) are oriented with respect to each other such that during operation of the device force or forces applied to the device through the single operating hand compress the chamber (26) so as to deliver a prescribed volume of gas to the respiratory tract of the individual, cause formation of a substantial seal between the delivery means (e.g., mask) and the maxillo-facial region of the individual during the compression of the chamber, and can selectively maintain or release the seal during refilling of the chamber.

The device can also include mechanisms for limiting the void volume of the chamber or for limiting the degree to which the chamber may be compressed (delivery-limiting), thus regulating the volume of gas delivered to the patient.

FIG.1

## A RESUSCITATION DEVICE

### FIELD OF THE INVENTION

This invention relates to manually-operated resuscitators and other devices for providing air or other appropriate gas or mixture of gases to the respiratory tract of an individual during emergency medical treatment. In particular, this invention relates to a single-hand resuscitation device for providing gas to the respiratory tract of an individual.

### BACKGROUND OF THE INVENTION

In many emergency medical situations it is necessary to assist the patient's breathing. When sophisticated respiratory instruments are not available, assistance is usually provided by a "portable resuscitator," "bag-valve-mask device" or "bag-mask ventilator." Prior art bag-mask resuscitators consist of a plastic or rubber face mask, a valve/conduit and a bag. The face mask conforms to the general contours of a human face, lips, teeth and/or gums (i.e., all or part of the maxillo-facial region) to facilitate fitting the mask to the patient. The valve/conduit is usually a generally L-shaped tube which is placed between the mask and the bag. The valve/conduit may contain one or more valves which regulate gas flow through the valve/conduit in both directions. The bag forms a compressible chamber which usually holds 1,000-1,600 ml of gas when filled. When the bag is compressed, gas passes out of the bag through the valve/conduit and to the patient through the mask. Prior art devices of this nature are typified by the Laerdal Portable Resuscitator, the Ambu® Universal Resuscitator MS-30 and the Hope II Resuscitator (all commercially available from Dynamed, Inc., 6200 Yarrow Drive, Carlsbad, California 92008, product nos. E09570, E21226 and E17236, respectively).

Two major problems have been associated with these traditional bag-mask resuscitators. First, because the health professional has only two hands, it is difficult to (1) maintain a tight seal between the mask and the patient's maxillo-facial region (2) maintain the patient's head and neck in the proper position with one hand, and (3) deliver gas to the patient by squeezing the bag with the other hand. When the patient is on his back and the head and neck are in their normal or "neutral" position the airway to the respiratory tract can become partially or wholly occluded by the tongue which falls backward against the posterior oropharynx. The neutral head/neck position also creates three angles along the airway which make gas movement to the lungs more difficult. To prevent occlusion by the tongue and to maximize free gas flow, the head and neck should be hyperextended, thus lifting the jaw, moving the tongue forward and creating only one angle for gas to travel around to reach the respiratory tract. Unfortunately, however, because the health professional is also trying to maintain a good seal between the mask and the patient's maxillo-facial region, he continually exerts a downward pressure against the maxillo-facial region, particularly the jaw. This downward force tends to push the patient's head and neck into a neutral or flexed position.

Health professionals have proposed several approaches to reconciling these opposing forces. The most popular approach is to "clamp" the mask to the patient's maxillo-facial region by positioning the thumb and one or two fingers over the mask, positioning the remaining fingers on the patient's jaw and "clamping down" to maintain a seal. Pressure can then also be exerted by the fingers on the jaw to hyperextend the head and neck. This approach is only partially effective because (1) the clamping hand becomes fatigued quickly and may not be able to maintain a proper seal, and (2) the hyperextension of the airway must be constantly re-evaluated because it is easy to allow the head and neck to return to the neutral position.

Another suggested approach uses the health professional's knees to hold the head and neck in the hyperextended position while using one hand to form a seal between the mask and the patient's maxillo-facial region. This solution is also only partially effective because it cannot be conveniently used where the patient is not on the ground or other surface onto which the health professional can climb to use his knees.

The second major problem associated with the traditional mask resuscitators is their inability to deliver a constant acceptable volume of gas to the patient. To adequately exchange carbon dioxide from the blood there must be a certain minimum volume of gas moving in and out of the lungs. Conversely, damage can be done to the patient's lungs if too much gas is delivered, especially when the patient is a smaller individual, e.g.. a child. The American Heart Association has established 800 ml as the standard volume of gas which should be delivered to an adult with each ventilation using a resuscitator. The traditional resuscitation device bags usually have a volume over one liter and are not calibrated to let the health professional know how much gas is being delivered. As a result, it is difficult to determine whether the patient is receiving too much or too little gas. Moreover, the bags are usually very large in circumference, making it difficult to squeeze out large volumes. Large bag size also leads to quick fatigue of the squeezing hand which can cause reduction in the volume of gas delivered to the patient. These problems become even more complicated when one tries to use the same device on both an adult and a child who each require a different specific volume of gas.

It would, therefore, be desirable to provide a resuscitation device which can be completely operated with a single hand, thus leaving the other hand free to maintain the patient's head and neck in a hyperextended position. It would also be desirable for such resuscitation device to reliably deliver a constant volume of gas determined by the health professional using the device.

## SUMMARY OF THE INVENTION

A resuscitation device is disclosed for delivering air or other appropriate gas or mixture of gases to the respiratory tract of a patient which can be utilized using only one hand to operate the device, leaving the other hand completely free to manipulate the patient's head and neck. The device comprises a housing defining a chamber, means for delivering gas from the chamber to the patient and means for filling the chamber with gas. The delivery means is usually a mask or a mask combined with a conduit connecting the chamber to the mask. The housing and delivery means are oriented with respect to each other such that during operation of the device force or forces applied to the device through the single operating hand selectively compresses the chamber so as to deliver a prescribed volume of gas to the respiratory tract of the individual, causes formation of a substantial seal between the delivery means (e.g., mask) and the maxillo-facial region of the individual during the compression of the chamber, and maintains or releases the seal during refilling of the chamber. Although the invention is, in some of the appended claims, defined in terms of operation with a single hand, it should be noted that the devices of the present invention can also be operated by other or multiple body parts (e.g., two hands, knees).

The device can also include means for limiting the void volume of the chamber or for limiting the degree to which the chamber may be compressed (delivery-limiting), thus regulating the volume of gas delivered to the patient.

## BRIEF DESCRIPTION OF THE FIGURES

Fig. 1 is an exploded view of the preferred embodiment of the device of the present invention.

Fig. 2 is a cutaway view of the preferred embodiment.

Fig. 3 depicts an embodiment of a volume limiting means for use with the devices of the present invention. Fig. 3 also demonstrated the single-hand operation of a device of the present invention.

Fig. 4 depicts another embodiment of a volume limiting means for use with the devices of the present invention.

Fig. 5 is a pictorial view of an alternative embodiment of the device of the present invention.

Fig. 6 is a sectional view of the alternative embodiment along line 6-6 shown in Fig. 5.

Figs. 7-11 depict certain other embodiments of volume limiting means for use with the devices of the present invention.

Figs. 12 and 13 depict certain embodiments of delivery limiting means for use with devices of the present invention.

## DETAILED DESCRIPTION OF THE INVENTION

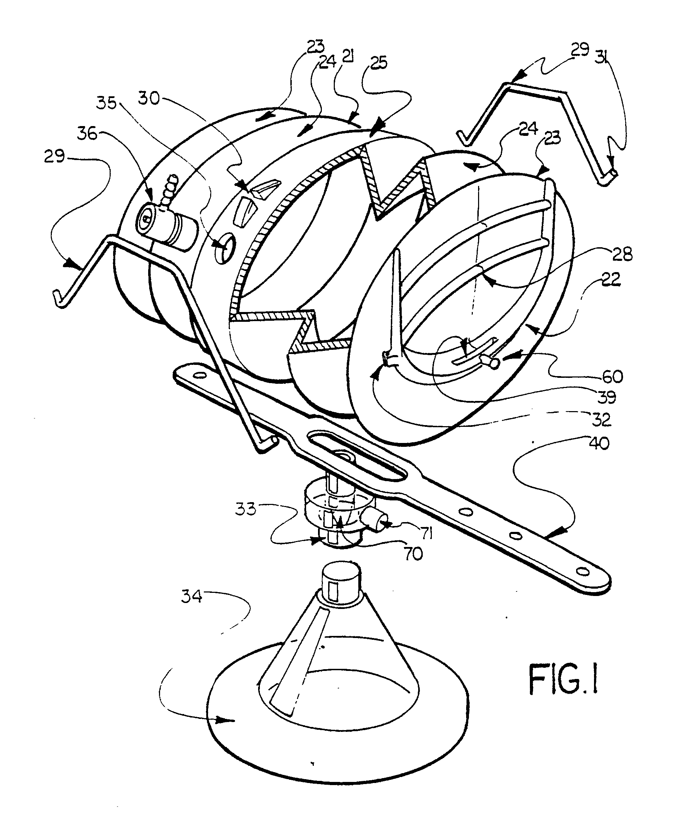

The preferred embodiment of the device of the present invention is depicted in Figs. 1-4. As shown in Fig. 1, the housing 21 in its unassembled and fully extended form is a hollow elongated "accordion-like" structure having a generally oval-shaped cross section and closed ends 22. The housing 21 is 8.2 inches long when fully extended and the cross sectional dimensions are 8.0 inches along the long axis and 5.6 inches along the short axis. Along its length, the housing 21 is sectioned into two end baffles 23, two intermediate baffles 24 and one central baffle 25. The central baffle 25 is 1.2 inches wide, the intermediate baffles 24 are each 1.5 inches wide, and the end baffles 23 are each 0.5 inches wide, with width being measured along the length of the housing 21. A grip 28 is molded into the surface of each closed end 22 of the housing 21. Although the housing 21 can be made from any flexible material which is substantially impermeable to gas, the housing 21 of the preferred embodiment was blow-molded from a thermoplastic, e.g., polyethylene.

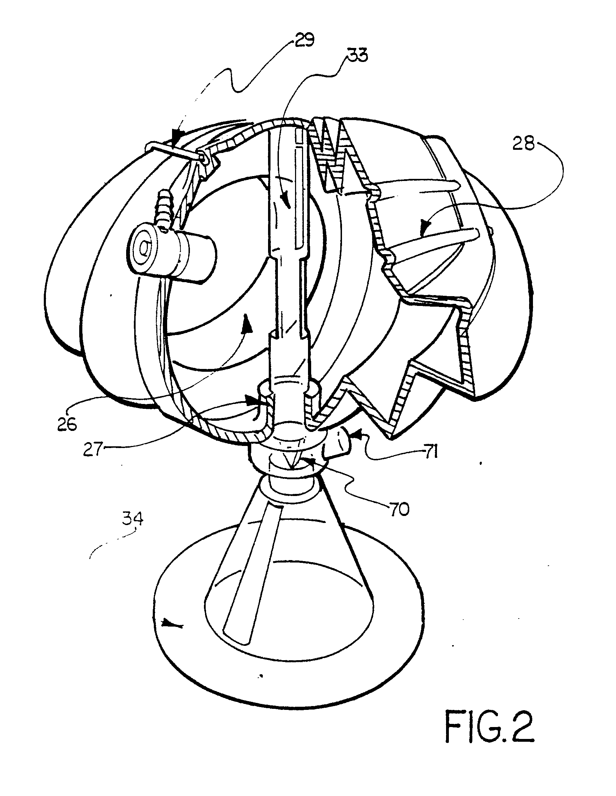

The housing 21 is hollow and defines a compressible chamber 26 as shown in Figs. 1 and 2. A delivery manifold 27 of cross sectional diameter 0.86 inches is located on one of the long sides of the central baffle 25 as shown in Fig. 2. The delivery manifold 27 extends through the housing 21 into the chamber 26 such that compression of the chamber 26 will cause gas to pass through the delivery manifold 27. As shown in Fig. 2, the chamber filling means of the preferred embodiment is a filling manifold 35 located on one of the short sides of the central baffle 25, the filling manifold 35 being fitted with a one-way valve 36 which only allows gas to pass into the chamber as it is refilled. Other means for filling the chamber with gas which are known to those skilled in the art but not specifically disclosed herein are intended to be included within the term "filling means" as that term is used in the appended claims so long as such filling means can be oriented with respect to the other elements of the devices of the present invention so as to allow single-handed delivery of gas to the patient and sealing with the patient's maxillo-facial region as described below and in the appended claims.

When the device 20 of the preferred embodiment is assembled, the housing 21 is partially compressed by two springs 29 into a bellows-like configuration. The springs 29 are crafted from heavy wire to have the general shape shown in Fig. 1. Starting at one end of the spring 29, the wire extends 0.1 inches, then turns 90 degrees to the right and extends 2.9 inches, then turns 58 degrees to the left and extends 1.3 inches, then turns 58 degrees to the left and extends 2.9 inches, and finally turns 90 degrees to the right and extends 0.1 inches to the other end of the spring 29. Both ends of each spring 29 have a hook 31.

To form the housing 21 into the bellows-like configuration, as shown in Fig. 2, one edge of the housing 21 is compressed and the generally right-angled segment of the springs 29 are placed over the edge of the housing 21 at two positions 30 such that (1) the compressed edge of the housing 21 remains compressed and (2) the free ends of the springs 29 extend away from the ends 22 of the housing 21. The hooks 31 at both ends of each spring 29 are then hooked over the end baffle 23 at four positions 32 (only one shown in Fig. 1). The form of the springs 29 holds the housing 21 open in a bellows-like configuration as shown in Fig. 2. The springs 29 also resist full compression of the housing 21 during use and pull the housing 21 into its "open" position when compressive forces are not being applied to the ends 22 of the housing 21.

The preferred embodiment has delivery means for delivering gas to the patient which includes a conduit 33 and a mask 34, A conduit 33 of diameter 0.86 inches and 6.0 inches long is connected to the housing 21, as shown in Fig. 2, such that gas passing through the delivery manifold 27 passes through the conduit 33. The conduit 33 may, but need not necessarily, contain valves and outlets for regulating the direction of gas flow through the conduit 33. The preferred embodiment incorporates a one-way valve 70 and an exhaust 71. The one-way valve 70 allows gas to pass from the chamber to the mask and prevents gas from passing from the patient's lungs back into the chamber. Gas leaving the patient's lungs is expelled through exhaust 71. These and other appropriate valves, outlets and their arrangement for these purposes are well-known to those skilled in the art.

The conduit 33 is attached at its other end to a mask 34, as shown in Fig. 2. Many masks traditionally used with resuscitation devices are appropriate and are commercially available (from, for example, DynaMed). Alternatively, the delivery means can include other means for introducing gas to the respiratory tract, e.g., an endotracheal tube or esophageal obturator airway. Other means for delivering gas to the patient which are known to those skilled in the art but not specifically disclosed herein are intended to be included within the term "delivery means" as that term is used in the appended claims so long as such delivery means can be oriented with respect to the other elements of the devices of the present invention so as to allow single-handed delivery of gas to the patient and sealing with the patient's maxillo-facial region as described below and in the appended claims.

All connections between the housing 21, conduit 33 and mask 34 are sealed to prevent gas from escaping.

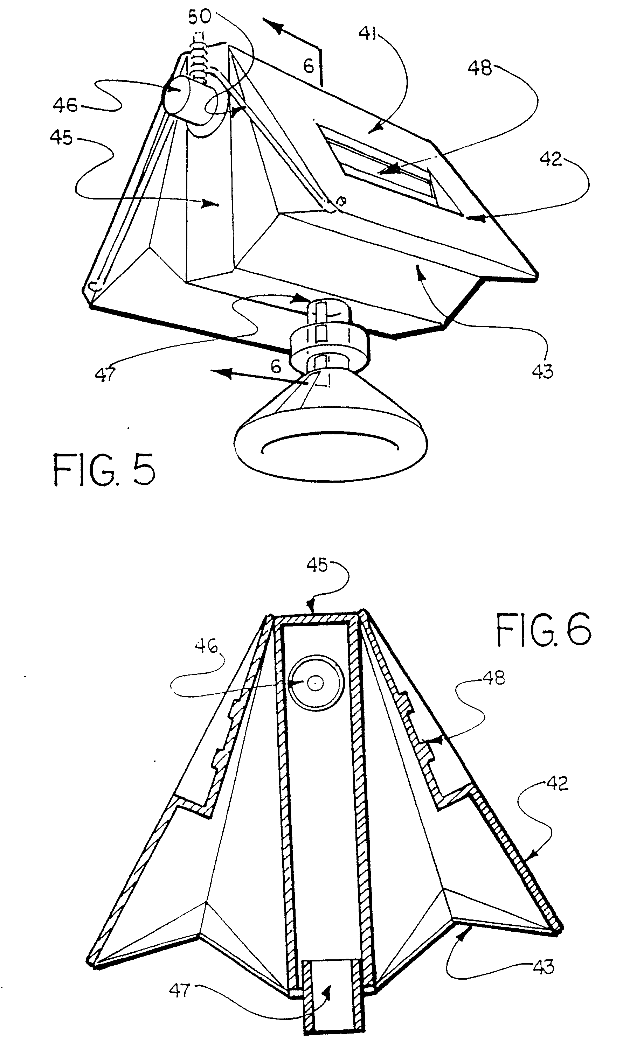

Figs. 5 and 6 depict an alternative embodiment of the housing of the device of the present invention. As shown in Fig. 5, the alternative housing 41 is, unlike the preferred embodiment, formed as a "true" bellows having two closed ends 42 of generally rectangular cross section having dimensions 8.0 inches by 5.0 inches. The ends 42 are each joined at one edge to a central baffle 45. The other edges of the ends 42 are each joined to an end baffle 43. The end baffles 43 are in turn joined to the central baffle 45. The central baffle 45 is 1.2 inches wide and the end baffles 43 are each 3.0 inches wide. A grip 48 is formed on the surface of each end 42 of the alternative housing 41. As shown if Figs. 5 and 6, the alternative housing 41 incorporates a delivery manifold 47 which is located on one of the long sides of the central baffle 45. Delivery means described above are also useful in this alternative embodiment.

As shown in Fig. 5, the alternative housing 41 also incorporates as filling means a filling manifold (not shown) and a filling valve 46 which is located on one of the short sides of the central baffle 45 similar to the corresponding structure in the preferred embodiment. Alternatively, the filling means in a device of the present invention can be located at other locations on the housing or at a location not on the housing (e.g., on the conduit leading from the chamber to the mask). When the filling means is not located on the housing, gas flow throughout the device should be properly regulated so as to the chamber is refilled with fresh gas and not with gas from the patient's lungs.

The alternative housing 41 does not, however, have two springs like those of the preferred embodiment. Compression of the housing is resisted by a single coiled spring 50 shown in Fig. 5. The spring 50 pushes the alternative housing 41 into the expanded position when no compressive forces are applied to the housing. Alternatively, the expanded position can be maintained by including some internal form (e.g., foam, sponge, etc.) inside the housing.

Although the alternative housing 41 can be made from any flexible material which is substantially impermeable to gas, the alternative housing 41 of this alternative embodiment was constructed from cardboard and other paper products.

Other forms for the housing in a device of the present invention, which will be apparent to those skilled in the art but are not specifically disclosed herein, are intended to be included within the term "housing" as that term is used in the appended claims so long as such housing can be oriented with respect to the other elements of the devices of the present invention so as to allow single-handed delivery of gas to the patient and sealing with the patient's maxillo-facial region as described below and in the appended claims.

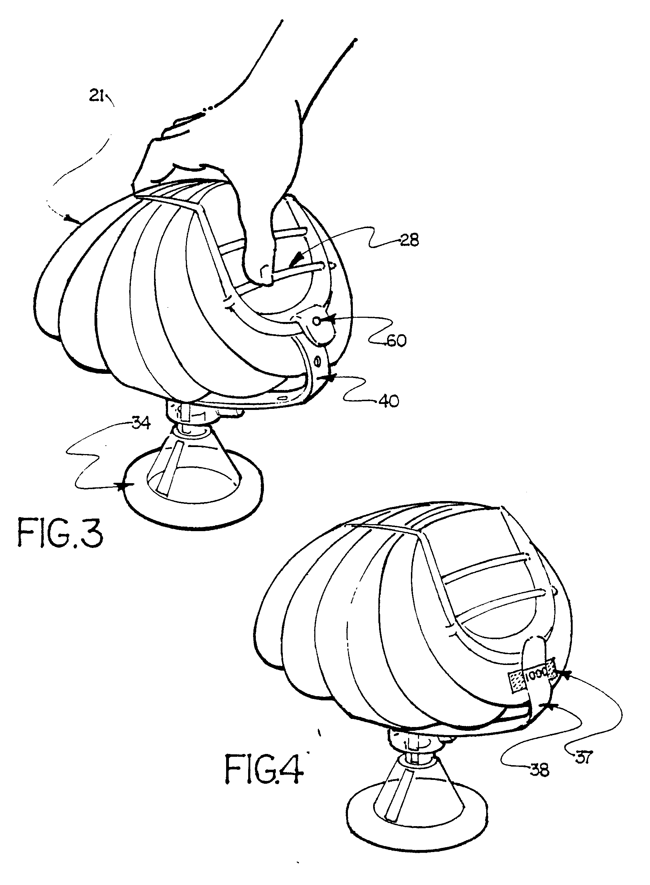

The preferred embodiment of the device 20 of the present invention is also fitted with means for restricting the amount of gas which fills the chamber and, as a result, is delivered to the patient when the chamber is compressed. Each such void-limiting means can be used with most any embodiment of the device of the present invention. Figs. 1, 3 and 4 show two ways of limiting the void volume of the expanded chamber. In Figs. 1 and 3, a punched strip 40 is attached at one end to one closed end of the housing and hangs free over most of the length of the strip. To limit the void volume of the chamber, the punched strip 40 is inserted through slot 39 in the other closed end of the housing and engaged with tab 60 on housing 21 holding the notched strip 40 in place. Each hole on the punched strip 40 corresponds to a different prescribed chamber void volume. Fig. 4 shows use of a velcro strip pair 37 and 38. One half of the velcro pair 37 is anchored over its entire length to one closed end of the housing. The other half of the velcro pair 38 is attached at one end to the other closed end of the housing and hangs free over most of the length of the strip. To limit the void volume of the chamber, the free-hanging strip 38 is engaged with the anchored strip 37, thus preventing the housing from expanding wider than the length of the segment of the free-hanging strip 38 extending between the ends. As more of the length of the free-hanging strip 38 is engaged with the anchored strip 37, the void volume of the chamber is decreased. The free-hanging strip 38 is marked at various places to indicate the void volume of the chamber allowed by engaging the velcro pair to that point on the free-hanging strip 38.

Fig. 3 depicts the preferred method of operating the device 10 of the present invention. The palm of a single hand may be placed along the compressed or top edge of the housing 21. The thumb and fingers grab the device along the grips 28. The mask 34 is then placed over the patient's maxillo-facial region. The single hand then applies force to the device to form a seal between the mask 34 and the maxillo-facial region. When a seal has been achieved, the same single hand then applies compressive forces, the directions of which are opposed to (i.e., in any direction other than in complete alignment with) the direction of the applied sealing force, to the ends 22 of the housing 21, thus compressing the chamber 26 and delivering the prescribed volume of gas through the mask 34 to the patient. Alter the chamber has been fully compressed, the single hand releases the compressive forces on the housing, thus allowing the chamber 26 to refill through the filling manifolds 35. The applied sealing forces may be selectively maintained, or fully or partially released during the refilling process in order to maintain or release, as desired, the seal against the maxillo-facial region of the patient and maintain positive end expiratory pressure. The ability to both hold the device in place to achieve a seal and deliver compressive forces to the chamber with a single hand is one of the features that distinguishes the devices of the present invention over the prior art.

The herein described embodiments of the devices of the present invention are also characterized by the fact that a virtually constant distance is maintained between the point on the device where the force is applied by the single hand and the patient's maxillo-facial region where that applied force results in formation of a seal. Although the embodiments described herein are characterized by that fact, the appended claims are not intended to be limited by such characteristic unless expressly stated in a given claim. Certain embodiments which are intended to be included within the coverage of some of the appended claims may not maintain such a constant distance. As long as a certain embodiment contains all of the elements and qualities described in an appended claim, that does not expressly require that such a constant distance be maintained, such embodiment is intended to be covered by such claim regardless of the fact that such a constant distance is not maintained in such embodiment.

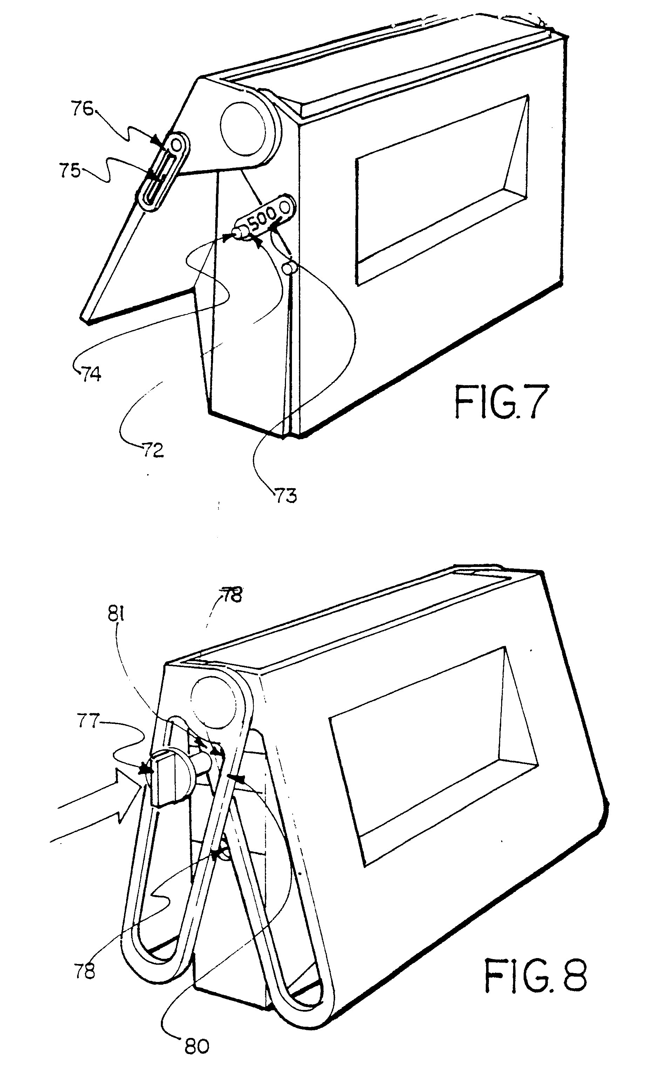

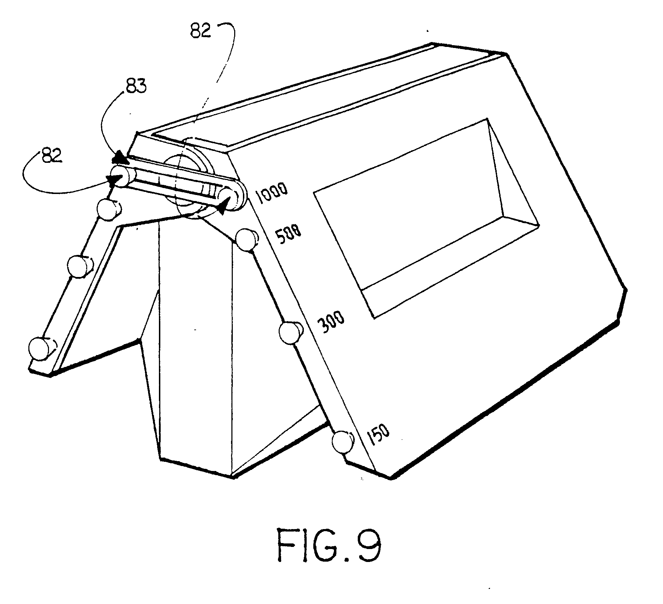

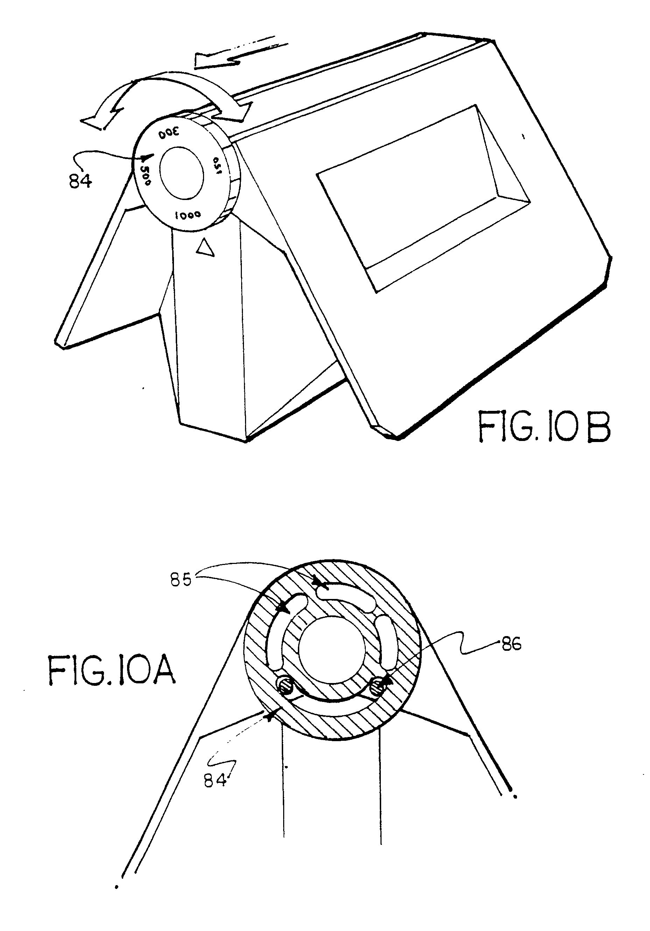

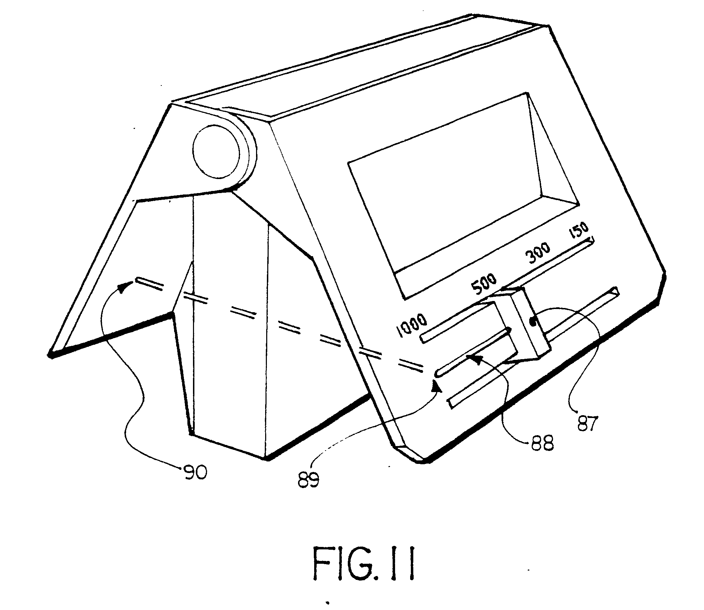

Figs. 7-11 show five other mechanisms for limiting the void volume of the expanded chamber. In Fig. 7, hole 72 of tab 73 is engaged with pin 74 to reduce the volume to which the chamber can expand. Alternatively or additionally, slot 75 of tab 76 is engaged with pin 74 to limit the void volume of the chamber. In the uncompressed state of the device, pin 74 will be positioned within slot 75 toward the free end of tab 76 such that that half of the chamber is prevented from fully opening. As the housing is compressed, pin 74 slides toward the other end of slot 75 until the void volume is completely delivered. In Fig. 8, pin 77 is engaged with holes 78 which correspond to different void volumes. As the housing tries to open up, pin 77 contacts ribs 80 and 81 preventing the housing from fully opening. In Fig. 9, pins 82 are engaged with clip 83 to prevent the chamber from fully expanding. The clip 83 can be moved to different sets of pins to vary the void volume. In Figs. 10a and 10b, knob 84 is fitted with four internal recesses 85 which correspond to four different void volumes. When the chamber is compressed, knob 84 is pulled out, turned and pushed or pulled back in such that the internal recess corresponding to the desired void volume becomes engaged with pins 86. When the compressing force on the chamber is released, the ends of the internal recess prevent the chamber from fully expanding. In Fig. 11, one end of the device is fitted with a sliding member 87 which is attached to one end of string 88. String 88 runs through hole 89 and the other end of string 88 is attached at point 90 to the opposite end of the device. Sliding member 87 is then adjusted to shorten or lengthen the length of string 88 extending between hole 89 and point 90 to decrease and increase, respectively, the allowed void volume of the chamber. These examples of void-limiting means for use with devices of the present invention are intended to be illustrative and not to limit the invention in any manner. Other void-limiting means may be used with the devices of the present invention and such other means are intended to fall within the scope of the invention.

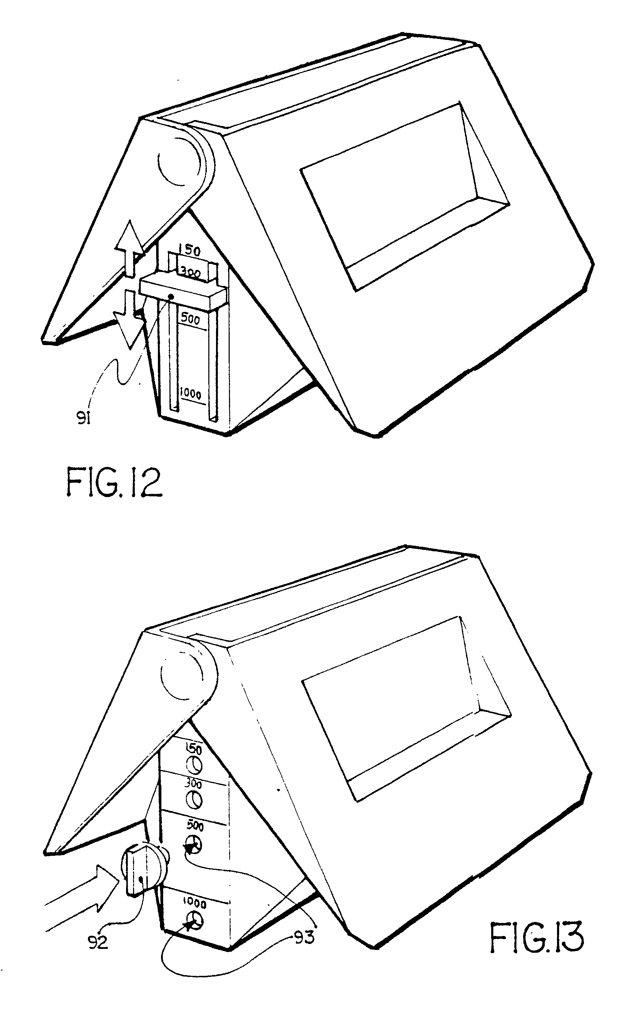

An embodiment of the present invention can also incorporate a mechanism for limiting the degree to which the housing and chamber can be compressed and, as a result, limit the volume of gas delivered to the patient. Figs. 12 and 13 show two possible delivery-limiting mechanisms for limiting the volume of gas delivered to the patient. In Fig. 12, sliding member 91 can be adjusted to various positions corresponding to various delivery volumes such that it blocks the complete compression of the chamber, thus delivering a limited volume of gas. In Fig. 13, pin 92 can be inserted in various holes 93 corresponding to various delivery volumes such that it blocks the complete compression of the chamber, thus delivering a limited volume of gas. Other delivery-limiting means may be used with the devices of the present invention and such other means are intended to fall within the scope of the invention.

From the foregoing, it will be obvious to those skilled in the art that various modifications in the above-described devices can be made without departing from the spirit and scope of the invention. Accordingly, the invention may be embodied in other specific forms without departing from the spirit or essential characteristics thereof. Present embodiments, therefore, are to be considered in all respects as illustrative and not restrictive, the scope of the invention being indicated by the appended claims rather than by the foregoing description, and all changes which come within the meaning and range of equivalency of the claims are therefore intended to be embraced therein.

## Claims

1. A resuscitation device for providing air or other appropriate gas or mixture of gases to the respiratory tract of an individual, said device comprising:
a housing defining a compressible chamber, said housing having at least one delivery manifold through which gas can pass when said chamber is compressed;
delivery means in communication with said delivery manifold such that when said chamber is compressed gas passes to said individual by way of said delivery means; and
filling means in communication with said chamber for filling said chamber with gas;
said housing and said delivery means being oriented with respect to each other such that during operation of said device force or forces applied to said device through a single hand operating said device can selectively (i) compress said chamber so as to deliver a prescribed volume of gas to said respiratory tract of said individual, (ii) cause formation of a substantial seal between said delivery means and the maxillo-facial region of said individual during said compression of said chamber or (iii) maintain or release said seal during compression or refilling of said chamber.

2. The resuscitation device of claim 1 wherein said delivery means comprises:
a mask portion in communication with said delivery manifold, such that gas passing from said chamber passes into said mask portion to said individual.

3. The resuscitation device of claim 1 wherein said delivery means comprises:
a conduit in communication with said delivery manifold; and

a mask portion in communication with said conduit, such that gas passing from said chamber into said conduit passes into said mask portion to said individual.

4. A resuscitation device for providing air or other appropriate gas or mixture of gases to the respiratory tract of an individual, said device comprising:

a housing defining a compressible chamber, said housing having at least one delivery manifold through which gas can pass when said chamber is compressed;

delivery means in communication with said delivery manifold such that when said chamber is compressed gas passes to said individual by way of said delivery means; and

filling means in communication with said chamber for filling said chamber with gas;

said housing and said delivery means being oriented with respect to each other such that during operation of said device (i) a virtually constant distance is maintained between a first point on said housing through which a force or forces are applied to said device by said single hand and a second point on the maxillo-facial region of said individual, and (ii) said force or forces can selectively maintain or release a substantial seal between said delivery means and the maxillo-facial region of said individual during said compression or refilling of said chamber.

5. The resuscitation device of claim 4 wherein said delivery means comprises:

a mask portion in communication with said delivery manifold, such that gas passing from said chamber passes into said mask portion to said individual.

6. The resuscitation device of claim 4 wherein said delivery means comprises:

a conduit in communication with said delivery manifold; and

a mask portion in communication with said conduit, such that gas passing from said chamber into said conduit passes into said mask portion to said individual.

7. A resuscitation device for providing air or other appropriate gas or mixture of gases to the respiratory tract of an individual, said device comprising:

a housing defining a compressible chamber, said housing having at least one delivery manifold through which gas can pass when said chamber is compressed;

delivery means in communication with said delivery manifold such that when said chamber is compressed gas passes to said individual by way of said delivery means; and

filling means in communication with said chamber for filling said chamber with gas;

said housing and said delivery means being oriented with respect to each other such that during operation of said device (i) sealing force or forces are applied by a single hand operating said device to said device so as to maintain a substantial seal between said delivery means and the maxillo-facial region of said individual during compression or refilling of said chamber, and (ii) compressive force or forces opposed to said sealing force or forces can be applied by said single hand to said device so as to compress said chamber and to deliver gas to said individual.

8. The resuscitation device of claim 7 wherein said delivery means comprises:

a mask portion in communication with said delivery manifold, such that gas passing from said chamber passes into said mask portion to said individual.

9. The resuscitation device of claim 7 wherein said delivery means comprises:

a conduit in communication with said delivery manifold; and

a mask portion in communication with said conduit, such that gas passing from said chamber into said conduit passes into said mask portion to said individual.

10. The resuscitation device of claims 1 2, 3, 4, 5, 6, 7, 8, or 9, said device additionally comprising:

resistance means in communication with said housing for returning said chamber to its uncompressed state.

11. The resuscitation device of claims 1 2, 3, 4, 5, 6, 7, 8, or 9, said device additionally comprising:

void-limiting means in communication with said housing for limiting to said prescribed volume the uncompressed void volume of said chamber.

12. The resuscitation device of clad 1 2, 3, 4, 5, 6, 7, 8, or 9, said device additionally comprising:

delivery-limiting means in communication with said housing for limiting to said prescribed volume the volume of gas passing out of said chamber when said chamber is compressed.

13. A resuscitation device for providing air or other appropriate gas or mixture of gases to the respiratory tract of an individual, said device comprising:

a housing defining a compressible chamber, said housing having at least one delivery manifold through which gas can pass when said chamber is compressed;

delivery means in communication with said delivery manifold such that when said chamber is compressed gas passes to said individual by way of said delivery means;

filling means in communication with said chamber for filling said chamber with gas; and

void-limiting means in communication with said housing for limiting to a prescribed volume the uncompressed void volume of said chamber.

14. A resuscitation device for providing air or other appropriate gas or mixture of gases to the respiratory tract of an individual, said device comprising:

a housing defining a compressible chamber, said housing having at least one delivery manifold through which gas can pass when said chamber is compressed;

delivery means in communication with said delivery manifold such that when said chamber is compressed gas passes to said individual by way of said delivery means;

filling means in communication with said chamber for filling said chamber with gas; and

delivery-limiting means in communication with said housing for limiting to a prescribed volume the volume of gas passing out of said chamber when said chamber is compressed.

FIG.1

FIG.2

FIG.3

FIG.4

FIG. 5

FIG. 6

FIG.7

FIG.8

FIG.9

84

300
.150
.500
1000

FIG. 10 B

85

86

84

FIG. 10A

FIG. 11

FIG.12

FIG.13

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 369 778 (NORRIS et al.) <br> * Column 2, lines 30-65; figures 1,2 * | 1,4,7, 10 | A 61 M 16/06 |
| Y | | 2,3,5,6 ,8,9,11 ,12 | |
| Y | FR-A-1 278 040 (COMMEINHES) <br> * Page 1, column 2, lines 8-13; figures 1,2 * | 2,3,5,6 ,8,9 | |
| Y | GB-A-1 550 720 (FABRYKA APARATURY RENTGENOWSKIEJ) <br> * Page 1, lines 33-42; figure 2 * | 11 | |
| Y | US-A-3 046 978 (LEA) <br> * Column 2, lines 44-48; column 3, lines 24-37; figures 1,3 * | 12 | |
| A | GB-A-1 190 877 (LIFE AND PRODUCTS) <br> * Page 2, lines 10-12,42-54; figure 2 * | 1,10 | |
| A | US-A-3 461 865 (CHOUINARD) <br> * Column 1, line 55 - column 2, line 8; column 2, line 43 - column 3, line 2; figure 1 * | 2,3,5,6 ,8,9,11 ,12 | **TECHNICAL FIELDS SEARCHED (Int. Cl.5)** <br><br> A 61 M |

~~The present search report has been drawn up for all claims~~

| Place of search | Date of completion of the search | Examiner |
|---|---|---|
| THE HAGUE | 28-05-1990 | SCHOENLEBEN J.E.F. |

EPO FORM 1503 03.82 (P0401)

European Patent

Office

| | **CLAIMS INCURRING FEES** |
|---|---|

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

| x | **LACK OF UNITY OF INVENTION** |
|---|---|

The Search Division considers that the present European patent application does not comply with the requirement of unity of invention and relates to several inventions or groups of inventions,

namely:

1. Claims 1-12: Resuscitation device especially provided for a single hand operation.

2. Claim 13: Resuscitation device not provided for a single hand operation.

3. Claim 14: Resuscitation device also not provided for a single hand operation.

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☒ None of the further search fees has been paid within the fixed time limit. The present European search report has been drawn up for those parts of the European patent application which relate to the invention first mentioned in the claims,

namely claims:  1-12